# EUROPEAN PATENT APPLICATION

(11) **EP 4 389 878 A1**
(43) Date of publication of application: **26.06.2024**
(21) Application number: 22858411.6
(22) Date of filing: 10.08.2022
(51) Int. Cl.: C12N 5/10, A61K 35/17, A61P 35/00, A61P 37/00, C12N 7/01, C12N 15/12, C12N 15/13, C12N 15/62

(54) **METHOD FOR PRODUCING GENE-MODIFIED T CELL POPULATION**

(30) Priority: 17.08.2021 JP 2021132969
(71) Applicant: Keio University, Tokyo, 108-8345 (JP); JSR Corporation, Tokyo 105-8640 (JP)
(72) Inventor: KAWAKAMI, Yutaka, Tokyo 160-8582 (JP); WAKUI, Seiki, Tokyo 105-8640 (JP); UENO, Masaru, Tokyo 105-8640 (JP); AOYAMA, Ryo, Tokyo 105-8640 (JP); SEKINE, Hitoshi, Tokyo 105-8640 (JP)
(74) Representative: TBK
(86) International application number: PCT/JP2022/030562
(87) International publication number: WO 2023/022090

(57) **Abstract**

Provided is a method of producing a gene-modified T cell population, including mixing a cell population containing T cells with beads each having bound thereto a virus containing a target gene to introduce the target gene into each of the cells of the cell population, wherein the cell population containing the T cells is cultured in a solution containing a CD3 signal activator that is present without being immobilized on a solid phase.

## Description

### Field of the Invention

The present invention relates to a method of producing a gene-modified T cell population, a gene-modified T cell produced by the method, and a use of the cell in disease treatment.

### Background of the Invention

Gene-modified T cells, such as a chimeric antigen receptor gene-modified T cell (CAR-T cell) and a TCR gene-modified T cell (TCR-T), have been attracting attention as innovative cancer therapeutic drugs for adoptive immunotherapy. The CAR-T cell has already been applied as a therapeutic drug for blood cancer in a clinical setting in our country.

In CAR-T cell therapy, typically, a T cell is collected from a patient, and a chimeric antigen receptor gene is introduced into the cell. The cell is cultured, and is then returned to the body of the patient. At this time, the efficiency with which the gene is introduced into the T cell is an important element involved in the outcome and cost of the treatment. In addition, the duration of the administered CAR-T cell in the body of the patient is also important for improving the outcome of the treatment. It has been conceived that as the T cell is a cell that is less differentiated, the cell functions in the body for a longer time period to improve the outcome of the treatment (Non-Patent Literatures 1 and 2).

In Patent Literature 1, there is a description of a virus-coated bead for efficient introduction of a gene into a cell, and there is also a description that after a T cell has been stimulated with magnetic beads having bound thereto an anti-CD3/anti-CD28 antibody, the cell is precoated with RetroNectin (trademark), and then the gene is introduced thereinto by using the virus-coated bead. In Patent Literature 2, there is a description that the efficiency with which a retrovirus is introduced into a cell is improved in the presence of a poloxamer having a molecular weight of from 12.8 kDa to about 15 kDa.

### Citation Listing

### Patent Literature

[Patent Literature 1] WO 2010/080032 A2
[Patent Literature 2] WO 2013/127964 A1

### Non-Patent Literature

[Non-Patent Literature 1] Nat Med, 2011; 17(10): 1290-1297
[Non-Patent Literature 2] Cancer Immunol Res, 2019, 7(5): 759-772

### Summary of the Invention

### Problems to be Solved by the Invention

To improve the effect of the treatment of a disease with a gene-modified T cell and to reduce cost therefor, it has been desired to efficiently produce a gene-modified T cell that maintains its undifferentiated state and has introduced there into a required gene.

### Means for Solving the Problem

The inventors of the present invention found that a gene-modified T cell maintaining its undifferentiated state was able to be efficiently produced by: treating a cell population containing T cells with a solution containing a CD3 signal activator; and then bringing the treated product into contact with beads each having bound thereto a virus containing a gene to be introduced.

Accordingly, the present invention provides the following.
[1] A method of producing a gene-modified T cell population, comprising
   mixing a cell population containing T cells with beads each having bound thereto a virus containing a target gene, and
   introducing the target gene into each of the cells of the cell population,
   wherein the cell population containing the T cells is cultured in a solution containing a CD3 signal activator that is present without being immobilized on a solid phase.
[2] The production method according to Item [1], wherein the cell population containing the T cells is whole blood, peripheral blood mononuclear cells, a cell population in a buffy coat solution, a cell population obtained by leukapheresis, or a culture thereof.
[3] The production method according to Item [1] or [2], wherein the CD3 signal activator is an anti-CD3 antibody.
[4] The production method according to Item [3], wherein the anti-CD3 antibody is OKT3.
[5] The production method according to any one of Items [1] to [4], wherein the solution containing the CD3 signal activator contains IL-2.
[6] The production method according to any one of Items [1] to [5], wherein the virus is at least one kind selected from the group consisting of: a retrovirus; a lentivirus; an adenovirus; an adeno-associated virus; and a simian virus.
[7] The production method according to any one of Items [1] to [6], wherein the beads are each coated with a molecule having abilities to bind to the T cells and the virus.
[8] The production method according to Item [7], wherein the molecule having abilities to bind to the T cells and the virus is at least one kind selected from the group consisting of: gelatin; collagen; elastin; fibronectin; pronectin; laminin; tenascin; fibrin; fibroin; entactin; thrombospondin; fragments thereof; variants thereof; and derivatives thereof.
[9] The production method according to Item [7], wherein the molecule having abilities to bind to the T cells and the virus is fibronectin, or is a fibronectin fragment or a fibronectin variant having a cell-adhesion domain and a heparin-binding domain of fibronectin.
[10] The production method according to any one of Items [1] to [9], wherein the beads are magnetic beads.
[11] The production method according to any one of Items [1] to [10], wherein the CD3 signal activator is free from being bound to the beads.
[12] The production method according to any one of Items [1] to [11], wherein the mixing of the cell population and the beads each having bound thereto the virus is performed in the presence of a polyoxyethylene-polyoxypropylene (POE/POP) triblock copolymer in which a content of a polyoxyethylene (EO) unit is 50% or more, and a polyoxypropylene (PO) unit has an average molecular weight of 900 or more.
[13] The production method according to Item [12], wherein in the polyoxyethylene-polyoxypropylene (POE/POP) triblock copolymer, the (PO) unit has an average molecular weight of from 1,700 to 4,500, and the content of the (EO) unit is 50% or more.
[14] The production method according to Item [12], wherein in the polyoxyethylene-polyoxypropylene (POE/POP) triblock copolymer, the (PO) unit has an average molecular weight of from 1,700 to 4,500, and the content of the (EO) unit is 70% or more.
[15] The production method according to any one of Items [1] to [14], wherein the target gene is a gene encoding a chimeric antigen receptor.
[16] The production method according to any one of Items [1] to [15], further comprising culturing the cells each having introduced thereinto the target gene to provide the gene-modified T cell population.
[17] The production method according to Item [16], wherein a time period from the culture of the cell population containing the T cells in the solution containing the CD3 signal activator to the culture of the cells each having introduced thereinto the target gene to provide the gene-modified T cell population is from 6 days to 21 days.
[18] The production method according to Item [16], further comprising recognizing, in each of the cells of the gene-modified T cell population, expression of at least one cell surface antigen selected from the group consisting of: CD45RA; CCR7; CD62L; CD73; CXCR3; CD27; CD28; KLRG1; IL7R; BACH2; TCF7; ID3; LEF1; FOXP1; KLF7; CD45RO; and CD95.
[19] A gene-modified T cell population, which is produced by the method of any one of Items [1] to [18].
[20] The gene-modified T cell population according to Item [19], wherein the gene-modified T cell population expresses at least one cell surface antigen selected from the group consisting of: CD45RA; CCR7; CD62L; CD73; CXCR3; CD27; CD28; KLRG1; IL7R; BACH2; TCF7; ID3; LEF1; FOXP1; KLF7; CD45RO; and CD95.
[21] A pharmaceutical composition for a cancer or an immune disease, comprising the gene-modified T cell population of Item [19] or [20] as an active ingredient.
[22] Use of the gene-modified T cell population of Item [19] or [20] for the production of a pharmaceutical composition for a cancer or an immune disease.
[23] The gene-modified T cell population according to Item [19] or [20], which is for use in the treatment of a cancer or an immune disease.
[24] A method of treating a cancer or an immune disease in a subject in need thereof, comprising a step of administering the gene-modified T cell population of Item [19] or [20] to the subject.

### Effects of the Invention

According to the present invention, the gene-modified T cell population can be efficiently produced. The gene-modified T cell population produced by the present invention may be used for the treatment of a disease in, for example, adoptive immunotherapy. Such cell population contains many T cells maintaining their undifferentiated states. Accordingly, the cell population can function in the body of a patient for a long time period, and hence can contribute to an improvement in effect of disease treatment and a reduction in cost therefor.

### Brief Description of Drawings

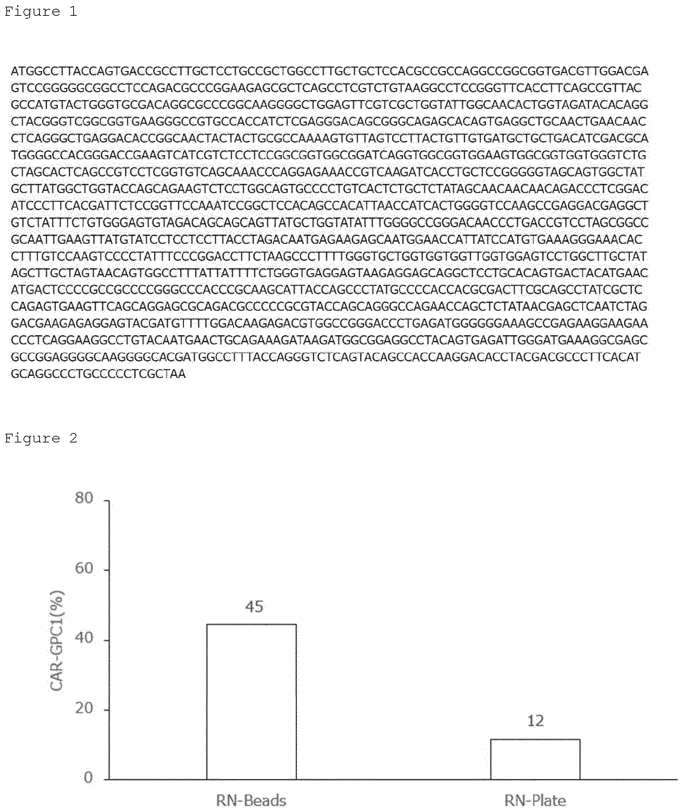

FIG. 1 shows the base sequence of a CAR-GPC1 gene.
FIG. 2 shows a comparison between the gene introduction efficiency of a plate method and that of a magnetic bead method based on the ratio of the number of CAR-GPC1-positive cells to the total number of living cells. The term "RN-Beads" refers to gene introduction with virus-bound RetroNectin (trademark)-coated magnetic beads (magnetic bead method), and the term "RN-Plate" refers to gene introduction with a virus-bound RetroNectin (trademark)-coated plate (plate method).

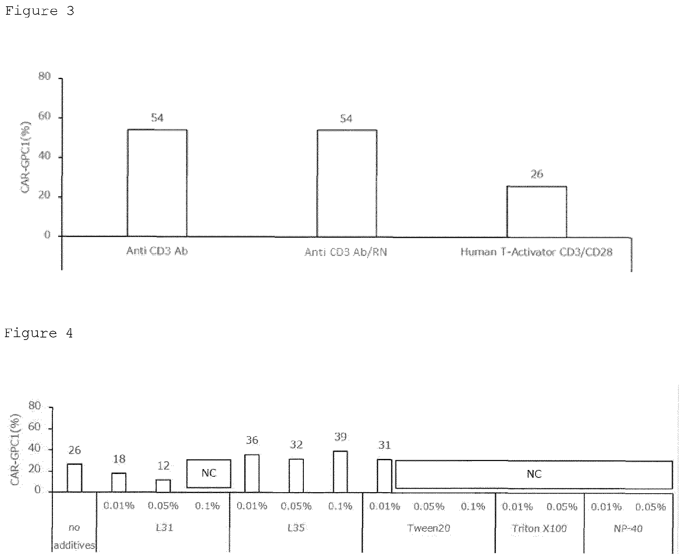

FIG. 3 shows a comparison among the influences of T cell activation methods in the magnetic bead method based on the ratio of the number of CAR-GPC1-positive cells to the total number of living cells. The term "Anti CD3 Ab" means a free anti-CD3 antibody, the term "Anti CD3 Ab/RN" means a RetroNectin (trademark)-coated plate and the free anti-CD3 antibody, and the term "Human T-Activator CD3/CD28" means anti-CD3 antibody-bound beads.
FIG. 4 shows a comparison among the influences of the addition of amphipathic molecules in the magnetic bead method based on the ratio of the number of CAR-GPC1-positive cells to the total number of living cells. The term "No additives" means that no amphipathic molecules are added, the term "L31" means that Pluronic (trademark) L31 is added, and the term "L35" means that Pluronic (trademark) L35 is added. The term "NC" means "Not Calculated" (unmeasurable due to cell death).

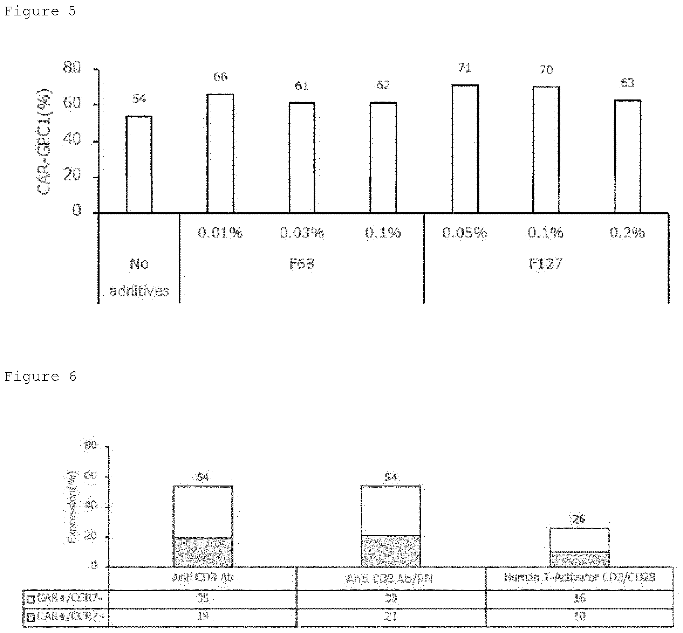

FIG. 5 shows a comparison among the influences of the addition of amphipathic molecules in the magnetic bead method based on the ratio of the number of CAR-GPC1-positive cells to the total number of living cells. The term "No additives" means that no amphipathic molecules are added, the term "F68" means that Pluronic (trademark) F68 is added, and the term "F127" means that Pluronic (trademark) F127 is added.
FIG. 6 shows a comparison among the influences of T cell activation methods in the magnetic bead method based on a ratio between the number of CAR-GPC1-positive and CCR7-negative (CAR+/CCR7-) cells and the number of CAR-GPC1-positive and CCR7-positive (CAR+/CCR7+) cells. A white part of a bar represents the ratio of the CAR+/CCR7- cells to the total number of living cells, a gray part of the bar represents the ratio of the CAR+/CCR7+ cells to the total number of the living cells, and numerical values above the bars each represent the total sum of the ratio of the CAR+/CCR7- cells and the ratio of the CAR+/CCR7+ cells. The term "Anti CD3 Ab" means a free anti-CD3 antibody, the term "Anti CD3 Ab/RN" means a RetroNectin (trademark)-coated plate and the free anti-CD3 antibody, and the term "Human T-Activator CD3/CD28" means anti-CD3 antibody-bound beads.

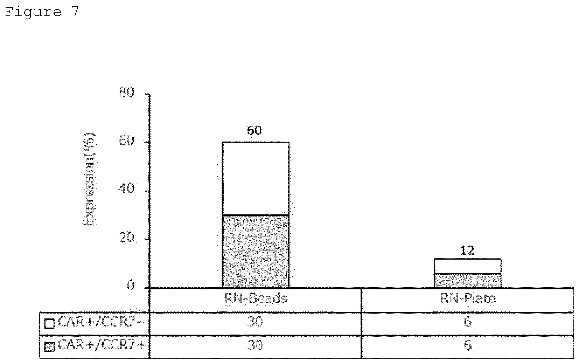

FIG. 7 shows a comparison between the magnetic bead method and the plate method based on a ratio between the number of CAR+/CCR7- cells and the number of CAR+/CCR7+ cells. A white part of a bar represents the ratio of the CAR+/CCR7- cells to the total number of living cells, a gray part of the bar represents the ratio of the CAR+/CCR7+ cells to the total number of the living cells, numerical values above the bars each represent the total sum of the ratio of the CAR+/CCR7- cells and the ratio of the CAR+/CCR7+ cells, the term "RN-Beads" means the magnetic bead method, and the term "RN-Plate" means the plate method.

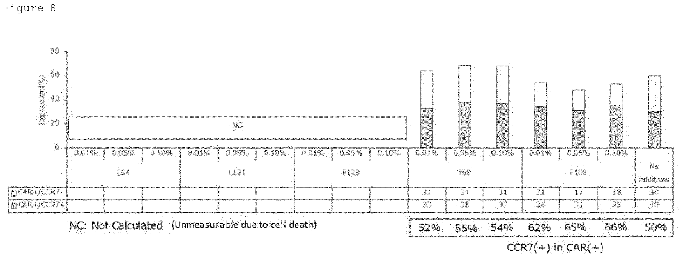

FIG. 8 shows a comparison among influences on the ratio of CCR7+ cells caused by a difference in added amphipathic molecule in the magnetic bead method based on a ratio between the number of CAR+/CCR7- cells and the number of CAR+/CCR7+ cells. The term "No additives" means that no amphipathic molecules are added, the term "L64" means that Pluronic (trademark) L64 is added, the term "L121" means that Pluronic (trademark) L121 is added, the term "P123" means that Pluronic (trademark) P123 is added, the term "F68" means that Pluronic (trademark) F68 is added, and the term "F108" means that Pluronic (trademark) F108 is added. The term "NC" means "Not Calculated" (unmeasurable due to cell death). A white part of a bar represents the ratio of the CAR+/CCR7- cells to the total number of living cells, and a gray part of the bar represents the ratio of the CAR+/CCR7+ cells to the total number of the living cells.

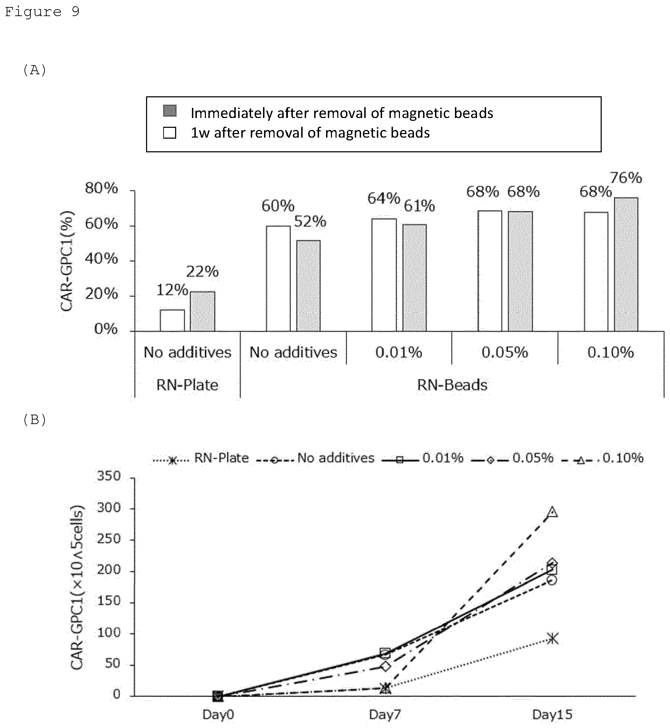

FIGS. 9 each show an influence of amphipathic molecules on the yield of CAR-GPC1-positive cells. FIG. 9(A) shows the ratio of the number of the CAR-GPC1-positive cells to the total number of living cells. The term "RN-Beads" means the magnetic bead method, and the term "RN-Plate" means the plate method. The term "No additives" means that no amphipathic molecules are added, and the numerical values "0.01%", "0.05%", and "0.1%" each mean that such amount of Pluronic (trademark) F68 is added. A gray part of a bar represents the ratio of the CAR-GPC1-positive cells immediately after the removal of the magnetic beads, and a white part of the bar represents the ratio of the cells after the residue has been cultured for 1 week after the removal of the magnetic beads. FIG. 9(B) shows the number of the CAR-GPC1-positive cells. The lines "RN-Plate" represent the results of the plate method (no amphipathic molecules are added), and the lines "No additives", "0.01%", "0.05%", and "0.1%" represent the results of the magnetic bead method in the presence of 0%, 0.01%, 0.05%, and 0.1% of Pluronic (trademark) F68, respectively.

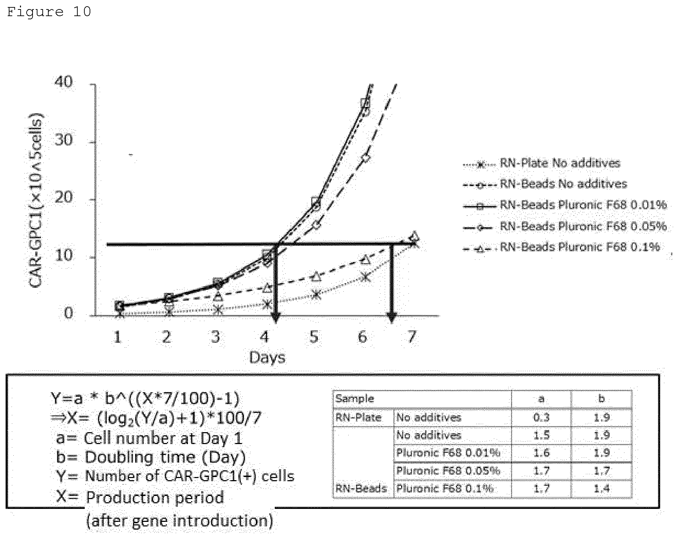

FIG. 10 shows a change in number of CAR-GPC1-positive cells with the number of culture days. The labels for the respective data in the figure are the same as those of FIG. 9(B).

### Description of Embodiments

All patent literatures, non-patent literatures, and other publications cited herein are incorporated herein by reference in their entirety.

The respective components listed herein may be used alone or in combination thereof unless otherwise stated.

In this description, an expression representing a numerical range such as "from A to B" is identical in meaning to "A or more and B or less," and hence A and B are included in the numerical range.

Proteins listed herein encompass mutant proteins obtained by the substitution, duplication, deletion, insertion, or frame shift of their amino acid sequences or base sequences encoding the amino acid sequences when the mutant proteins have the equivalent functions as those before the mutation.

The term "foreign" as used herein for the function, property, or trait of a cell is used for representing a function, a property, or a trait that is not originally present in the cell but is introduced from the outside. For example, a foreign gene or a foreign polynucleotide is a gene or a polynucleotide introduced from the outside into the cell. The foreign gene or the foreign polynucleotide may be derived from the same kind of living organism as that of the cell into which the gene or the polynucleotide has been introduced, or may be derived from a different kind of living organism (i.e., a different kind of gene or polynucleotide).

<Method of producing Gene-modified T Cell Population>

In one aspect, the present invention provides a method of producing a gene-modified T cell population. In one embodiment, the method comprises mixing a cell population containing T cells with beads each having bound thereto a virus containing a target gene to introduce the target gene into each of the cells of the cell population. In this case, the cell population containing the T cells is cultured in a solution containing a CD3 signal activator that is present without being immobilized on a solid phase. In another embodiment, the method comprises: culturing a cell population containing T cells in a solution containing a CD3 signal activator that is present without being immobilized on a solid phase; and mixing the cultured cell population with beads each having bound thereto a virus containing a target gene to introduce the target gene into each of the cells of the cell population.

### (T Cell Activation)

Examples of the cell population containing the T cells, which is used in the method of the present invention, include whole blood, peripheral blood mononuclear cells (PBMCs), a cell population in a buffy coat solution, a cell population obtained by leukapheresis, and cultures thereof. Each of those cell populations may be prepared from blood collected from a human or an animal, or is commercially available. The cell population containing the T cells to be used in the present invention is preferably a cell population derived from a human or a culture thereof.

The cell population containing the T cells is cultured in the presence of a CD3 signal activator. Thus, the T cells in the cell population are activated to proliferate. The CD3 signal activator is, for example, a CD3 agonist, which binds to CD3 on the surface of each of the T cells to activate the cell, such as an anti-CD3 antibody (e.g., an anti-CD3 antibody OKT3, an anti-CD3 antibody UCHT1, or an anti-CD3 antibody HIT3a), and such antibody is commercially available.

In the method of the present invention, the CD3 signal activator to be used at the time of the culture of the cell population containing the T cells is not immobilized on a solid phase. That is, the CD3 signal activator is not used under the state of being immobilized on a solid-phase carrier, such as a bead or a plate. The solution containing the CD3 signal activator (in which the CD3 signal activator is present under a free state without being immobilized on a solid phase) is preferably used. In a preferred embodiment, the CD3 signal activator is added to a culture solution for culturing the cell population containing the T cells. The concentration of the CD3 signal activator in the culture solution for culturing the cell population containing the T cells may be appropriately adjusted in accordance with the kind of the activator. For example, when the activator is an anti-CD3 antibody, the activator is used at a concentration in the range of preferably from 1 ng/mL to 1,000 ng/mL, more preferably from 1 ng/mL to 500 ng/mL, still more preferably from 1 ng/mL to 100 ng/mL.

The culture solution for culturing the cell population containing the T cells may be prepared in accordance with the composition of a culture solution to be typically used in the culture of the T cells. The culture solution is, for example, a serum-containing medium. The culture solution preferably contains interleukin-2 (IL-2). Further, the culture solution may contain any other T cell activator such as an anti-CD28 antibody. A medium for the activation and proliferation of the T cells is commercially available. Although the conditions under which the cell population containing the T cells is cultured only need to follow an ordinary method, the culture is preferably performed, for example, at 37°C and 5% CO₂ for from 2 days to 10 days.

### (Gene Introduction)

Next, in the method of the present invention, a gene is introduced into each of the cells cultured by the above-mentioned procedure. The mixing of the cells with a virus containing a target gene to be introduced (hereinafter referred to as "target gene") enables the introduction of the target gene into each of the cells. In the present invention, the target gene is introduced into each of the cells of the cell population, which has been cultured by the above-mentioned procedure, by mixing the cell population with the beads each having bound thereto the virus containing the target gene. When the virus is bound to each of the beads, and is then mixed with the cells, the efficiency with which the virus and each of the cells are brought into contact with each other is improved, and hence the efficiency with which the target gene is introduced into each of the cells can be improved.

Examples of the virus to be used in the method of the present invention include a retrovirus, a lentivirus, an adenovirus, an adeno-associated virus, and a simian virus. Those viruses may be used alone or in combination thereof. Of those, a retrovirus is preferably used.

The virus is bound to a carrier bead. The manner in which the virus and the bead are bound to each other is preferably adsorption, and examples thereof include, but not particularly limited to, a covalent bond, a chemical bond, a hydrophilic/hydrophobic interaction, and an electrostatic bond. The virus and the bead may be bound to each other through a protein such as BSA, or may be directly bound to each other. A bead to be used in the separation or collection of a sample in a biochemical field may be used as the bead. For example, a bead of any type suitable for binding to the virus, such as a magnetic bead, a silica bead, a dextran bead, a polylactic acid bead, a latex bead, or a polystyrene bead, may be used as the bead. To facilitate the separation of the bead having bound thereto the virus and the cells after the introduction of the gene, a magnetic bead is preferred as the bead. Examples of the magnetic bead include: fine beads each formed of a magnetic material selected from the group consisting of: triiron tetraoxide (Fe₃O₄); diiron trioxide (γ-Fe₂O₃); various ferrites; metals, such as iron, manganese, nickel, cobalt, and chromium; and alloys each containing two or more kinds of those metals; and magnetic beads each formed of a resin containing such magnetic material. Examples of the resin include a hydrophobic polymer and a hydrophilic polymer (see WO 2021/193802 A1). The particle diameter of the bead is preferably from 1 µm to 5 µm.

When the beads are each coated with a biocompatible molecule as described later, the beads are each preferably capable of adhering to the biocompatible molecule. For example, each of the beads may have a surface containing, as a material therefor, a substance that can physically adsorb the biocompatible molecule, or may have, on its surface, a functional group that can bind to the biocompatible molecule. Examples of the functional group include, but not limited to, a carboxyl group, an epoxy group, and a tosyl group. The particle diameter of each of the beads is preferably from 1 µm to 5 µm. Such beads are commercially available (e.g., Magnosphere (trademark) MX200/Carboxyl available from Medical & Biological Laboratories Co., Ltd.; Dynabeads (trademark) M-450 Epoxy available from Veritas Corporation; and FG beads COOH beads available from Tamagawa Seiki Co., Ltd.).

In the method of the present invention, it is preferred that each of the beads be free of the CD3 signal activator bound thereto (i.e., be a CD3 signal activator unbound type). It is more preferred that each of the beads be a CD3 signal activator unbound type, and be free of any other T cell activator such as an anti-CD28 antibody bound thereto.

To improve an adhesive property between the beads and the cells, to thereby improve the efficiency with which the gene is introduced into each of the cells, the beads to each of which the virus is bound are each preferably coated with a biocompatible molecule in advance. The biocompatible molecule preferably has abilities to bind to the T cells and the virus. Examples of such biocompatible molecule include biologically derived cell-adhesion proteins, such as gelatin, collagen, elastin, fibronectin, pronectin, laminin, tenascin, fibrin, fibroin, entactin, and thrombospondin, and fragments thereof, variants thereof, or derivatives thereof. Those proteins, fragments, variants, and derivatives may be used alone or in combination thereof. A more preferred example of the biocompatible molecule is fibronectin. In addition, a fragment of fibronectin or a variant thereof having the cell-adhesion domain and heparin-binding domain of fibronectin is also a more preferred example of the biocompatible molecule.

The biocompatible molecule is preferably derived from the same kind of living organism as that of each of the cells into which the target gene is introduced. For example, when a modified human T cell is produced, a human-derived biocompatible molecule, such as human fibronectin, or a fragment or variant thereof, is preferred. A human fibronectin fragment such as RetroNectin (trademark) (Takara Bio Inc.) may be used as a preferred example.

To coat each of the beads with the biocompatible molecule, a protocol suitable for the respective beads only needs to be followed. In the case of, for example, beads for physical adsorption each having a hydrophobic surface, the following only needs to be performed: a buffer solution containing the beads is mixed with the biocompatible molecule; and the mixed liquid is incubated while being shaken so that the beads do not aggregate. The mixed liquid is incubated at a temperature of preferably from 4°C to 37°C, more preferably from 20°C to 30°C for preferably from 30 minutes to 48 hours, more preferably from 12 hours to 24 hours. Next, the biocompatible molecule that is unreacted only needs to be removed by washing.

Before the virus is bound to each of the beads, the beads are each preferably blocked with a protein such as BSA. Each of the beads and the virus only need to be bound to each other by: mixing a solution containing the virus with the beads (that are each coated with the biocompatible molecule as required); and incubating the mixed liquid while shaking the mixed liquid so that the beads do not aggregate. The mixed liquid is incubated at a temperature of preferably from 4°C to 37°C, more preferably from 20°C to 35°C for preferably from 30 minutes to 24 hours, more preferably from 1 hour to 3 hours.

### (Target Gene)

Any gene may be selected as the target gene in the virus, which is to be introduced into each of the cells (target gene), in accordance with the applications of gene-modified T cells to be produced. The target gene may be a gene derived from the same kind of living organism as that of each of the cells into which the gene is introduced, may contain a different gene derived from a different kind thereof, or may be a chimeric gene containing genes derived from the same kind thereof and a different kind thereof. For example, when the gene-modified T cells are used in the CAR-T cell therapy of a cancer, the target gene is preferably a gene encoding a chimeric antigen receptor (CAR). The CAR is typically a polypeptide including an extracellular domain and an intracellular domain linked to each other through a transmembrane domain. The extracellular domain functions as a molecule that binds to a cell surface antigen specific to a target cancer, preferably an antibody molecule specific to the antigen. Preferred examples of the extracellular domain include an antigen-binding fragment of an anti-CD19 antibody and an antigen-binding fragment of an anti-glypican-1 (GPC-1) antibody. The intracellular domain is preferably a molecule that transfers a signal into a T cell to activate the T cell when the extracellular domain binds to the antigen. The intracellular domain is preferably a molecule that forms a complex with a T cell receptor (TCR) to produce an activation signal, and an example thereof is a CD3ζ intracellular domain.

In addition, for example, when the gene-modified T cells are used in the CAR-T cell therapy of an antibody-mediated autoimmune disease, the target gene contains a gene encoding a molecule binding to a B cell antigen receptor (BCR) that recognizes the antigen of an autoantibody responsible for the disease. Such target gene is, for example, a gene encoding a CAR containing desmoglein 3 (Science, 2016, 353, 6295: 179-184).

A preferred example of the target gene to be used in the present invention is a gene disclosed in WO 2016/208754 A1, the gene encoding a CAR. A more specific example thereof is a gene (SEQ NO: 1; eLife, 2020, 9, e49392) encoding a CAR formed of a CD8α leader sequence, the antibody recognition sequences of an anti-GPC-1 monoclonal antibody (e.g., VL and VH regions), a CD28 transmembrane domain, and a CD3ζ intracellular domain in the stated order from its N-terminus.

The target gene may be a single-stranded DNA, a double-stranded DNA, a single-stranded RNA, a double-stranded RNA, or any other artificial nucleic acid, and may be appropriately selected in accordance with the kind of the virus to be used.

The beads each having bound thereto the virus are mixed with the cell population cultured together with the CD3 signal activator described above. In the method of the present invention, the mixing and culture of the cells and the beads for the introduction of the gene may be performed in accordance with a typical procedure for transformation by a virus as long as a virus, which uses the cell population cultured together with the CD3 signal activator described above as a host, and is bound to a CD3 signal activator unbound type bead, is used. The mixing and the culture may be performed in accordance with, for example, a method disclosed in WO 2010/080032 A2. In one embodiment, the beads each having bound thereto the virus are dispersed in the culture solution in which the cell population has been cultured. Alternatively, the cell population and the beads each having bound thereto the virus may be added to and mixed in a newly prepared culture solution in an arbitrary order. When the culture solution containing the cell population and the beads each having bound thereto the virus is further cultured, the target gene in the virus is introduced into each of the cells.

After that, the expansion culture of the cells is performed. Although conditions for the culture only need to follow an ordinary method, the culture is preferably continued under the conditions of 37°C and 5% CO₂ until a required cell number is reached, preferably for 4 or more days from the gene introduction. In the process of the culture, the beads used in the gene introduction are appropriately removed from the culture. In the case of, for example, magnetic beads, the beads only need to be magnetically collected from the culture.

The gene introduction and the subsequent cell culture are preferably performed in the presence of an amphipathic molecule because the yield of the gene-introduced cells can be further increased. In one embodiment, the following may be performed: the amphipathic molecule is added to the culture solution after the culture of the cell population, and the beads each having bound thereto the virus are dispersed and cultured therein. In one embodiment, the following may be performed: a culture solution containing the amphipathic molecule is prepared, and the cell population and the beads each having bound thereto the virus are added to and cultured in the solution. However, an approach to adding the amphipathic molecule is not limited thereto.

In another embodiment, the virus may be bound to each of beads each having bound thereto the amphipathic molecule. Such amphipathic molecule-bound beads may be prepared in accordance with a protocol suitable for the respective beads. In the case of, for example, beads for physical adsorption each having a hydrophobic surface, the following only needs to be performed: a buffer solution containing the beads (that are each coated with the biocompatible molecule as required) is mixed with the amphipathic molecule; and the mixed liquid is incubated while being shaken so that the beads do not aggregate.

Although the amphipathic molecule to be used in the present invention is not particularly limited as long as the molecule has both of a hydrophilic group and a hydrophobic group, the molecule is, for example, a polyoxyethylene-polyoxypropylene (POE/POP) block copolymer, preferably a (POE/POP) block copolymer that is diblock or more, more preferably a (POE/POP) block copolymer that is triblock or more. A (POE/POP) triblock copolymer is still more preferred. The content (molecular weight ratio) of a polyoxyethylene (EO) unit in the (POE/POP) triblock copolymer is preferably 50% or more, more preferably 70% or more, still more preferably 80% or more. In addition, the average molecular weight of a polyoxypropylene (PO) unit in the (POE/POP) triblock copolymer is preferably 900 or more, more preferably from 900 to 5,000, still more preferably from 900 to 4,500, still more preferably from 1,700 to 4,500.

Preferred examples of the (POE/POP) triblock copolymer include: a (POE/POP) triblock copolymer in which the average molecular weight of its (PO) unit is 1,700 or more, preferably from 1,700 to 4,500, and the content of its (EO) unit is 50% or more; a (POE/POP) triblock copolymer in which the average molecular weight of its (PO) unit is 900 or more, preferably from 900 to 4,500, more preferably from 1,700 to 4,500, and the content ratio of its (EO) unit is 70% or more; a triblock copolymerization-type nonionic surfactant represented by CAS RN 9003-11-6 in which the average molecular weight of its (PO) unit is preferably from 900 to 4,500, more preferably from 1,700 to 4,500, and the content ratio of its (EO) unit is 70% or more; and a (POE/POP) triblock copolymer having a weight-average molecular weight of 8,400 and a POE weight ratio of 80%. An amphipathic molecule having such structure is commercially available, and there may be used a poly(ethylene glycol)-block-poly(propylene glycol)-block-poly(ethylene glycol) (CAS RN: 9003-11-6) sold under any one of, for example, the product names "Pluronic (trademark) F68," "Pluronic (trademark) F108," "Pluronic (trademark) F127," and "Pluronic (trademark) L35" (each manufactured by BASF Corporation), in which the average molecular weight of its (PO) unit is from 900 to 4,500, preferably from 1,700 to 4,500, and the content ratio (molecular weight ratio) of its (EO) unit is 50% or more, preferably 70% or more. The concentration of the amphipathic molecule to be used falls within the range of preferably from 0.01 mass% to 0.2 mass%, more preferably from 0.01 mass% to 0.1 mass% in terms of concentration in the culture solution containing the cell population and the beads each having bound thereto the virus.

In the method of the present invention, the gene-modified T cell population having introduced thereinto the target gene can be produced in high yield, and as a result, a required amount of gene-modified T cells can be produced in a shorter time period. In the method of the present invention, a time period required for the preparation of an amount of the gene-modified T cell population required for CAR-T cell therapy (i.e., a time period from the activation of the cell population containing the T cells to the proliferation of the cells after the gene introduction treatment to the required amount) is preferably 6 or more days. Meanwhile, the time period is preferably 21 or less days, more preferably 20 or less days, still more preferably 19 or less days, still more preferably 18 or less days, still more preferably 17 or less days, still more preferably 16 or less days, still more preferably 15 or less days, still more preferably 14 or less days, still more preferably 13 or less days.

The gene-modified T cells to be produced in the present invention are foreign gene-introduced T cells each having introduced thereinto the target gene. Whether or not the cells after the culture each have the target gene can be recognized by subjecting the cells to genome analysis by a PCR, sequencing, or the like, or examining the expression of a protein, which is encoded by the target gene, by the cells.

The fact that the cell population after the culture contains memory T cells maintaining their undifferentiated states can be recognized by recognizing, for the cell population, the expression of at least one selected from the group consisting of: CD45RA; CCR7; CD62L; CD73; CXCR3; CD27; CD28; KLRG1; IL7R; BACH2; TCF7; ID3; LEF1; FOXP1; KLF7; CD45RO; and CD95. The expression of at least one selected from the group consisting of: CCR7; CD45RA; and CD62L is preferably recognized. The expression of at least one selected from the group consisting of: CCR7; and CD45RA is more preferably recognized. The expression of CCR7 is still more preferably recognized. When the expression of CD45RA, CCR7, CD62L, CD73, CXCR3, CD27, CD28, KLRG1, IL7R, BACH2, TCF7, ID3, LEF1, FOXP1, or KLF7 is positive (+), or when the expression of CD45RO or CD95 is negative (-), it is recognized that the cell population contains the memory T cells maintaining their undifferentiated states.

The gene-modified T cell population produced by the method of the present invention contains preferably 10% or more, more preferably 15% or more, still more preferably 20% or more, still more preferably 30% or more of CCR7+T cells with respect to the total number of its cells.

### <Disease Treatment with Gene-modified T Cell Population>

An increased yield of the gene-modified T cells produced by the method of producing a gene-modified T cell population according to the present invention (hereinafter, "method of the present invention") described above or an improved undifferentiated state of each of the cells means that the present invention reduces production cost for gene-modified T cells useful in disease treatment, shortens a production period therefor, and improves the persistence of the effects thereof to contribute to a reduction in cost of disease treatment with the gene-modified T cells or an improvement in outcome of the treatment.

Accordingly, the present invention also relates to a gene-modified T cell population produced by the method of the present invention, and a use thereof in the treatment of a disease. In a preferred embodiment, the disease is a cancer or an immune disease.

In one aspect, the present invention provides a pharmaceutical composition for a cancer or an immune disease, comprising the gene-modified T cell population produced by the method of the present invention.

In another aspect, the present invention provides a use of the gene-modified T cell population produced by the method of the present invention, for the production of a pharmaceutical composition for a cancer or an immune disease.

In another aspect, the present invention provides a gene-modified T cell population produced by the method of the present invention, wherein the gene-modified T cell population is for use in the treatment of a cancer or an immune disease.

In another aspect, the present invention provides a method of treating a cancer or an immune disease in a subject in need thereof, comprising a step of administering the gene-modified T cell population produced by the method of the present invention to the subject.

The "cancer" to be treated with the gene-modified T cell population of the present invention refers to malignant tumors in general, and may be a liquid tumor or a solid tumor. In addition, the "cancer" may encompass various kinds of cancers, such as leukemia, a malignant lymphoma, epithelial cancer, an adenocarcinoma, a sarcoma, a neuroma, and a glioma, without being limited by its occurrence site (tissue and organ).

The "immune disease" to be treated with the gene-modified T cell population of the present invention is, for example, an antibody-mediated autoimmune disease such as pemphigus vulgaris.

The subject to which the gene-modified T cell population or pharmaceutical composition of the present invention is administered refers to a human and a nonhuman animal that require the treatment of a cancer or an immune disease. The subject is preferably a human patient having a cancer or an immune disease.

In a preferred embodiment, the pharmaceutical composition provided in the present invention is a drug for CAR-T cell therapy. The pharmaceutical composition is preferably an injection preparation, and may be administered into the body of the subject by being dripped thereinto through an injection or a catheter. The pharmaceutical composition comprises the gene-modified T cell population of the present invention, and may further comprise a pharmaceutically acceptable carrier as required. Examples of the pharmaceutically acceptable carrier include, but not limited to, water, oil, a buffering agent, phosphate buffered saline (PBS), and any other diluent for an injection. The composition of the present invention may comprise a pharmaceutically acceptable substance selected from the group consisting of: a lubricant; a binder; a wetting agent; an emulsifying agent; a pH adjuster; an isotonic agent; a buffering agent; an antioxidant; a suspending agent; a solubility improver; a preservative; a chelating agent; and the like as required. The pharmaceutically acceptable carrier and substance are known in the art (see, for example, Remington's Pharmaceutical Sciences, by E.W. Martin, Mack Publishing Co., Easton, Pa., 19th Edition, 1995).

The pH of the pharmaceutical composition is adjusted within a range having compatibility to an animal body, preferably to a pH of from 5.5 to 10, more preferably to a pH of from 5.5 to 8, still more preferably to a pH of from 5.5 to 7.5. The pH of the pharmaceutical composition may be adjusted with, for example, a pH adjuster or a buffer solution.

In one embodiment, the pharmaceutical composition is a one-pack type preparation comprising the gene-modified T cell population and the carrier. In another embodiment, the pharmaceutical composition is a two-pack type preparation separately comprising the gene-modified T cell population and a diluent or the like to be administered together.

The gene-modified T cell population of the present invention only needs to be administered in an effective dose to the subject. The term "effective dose" as used herein refers to an amount enough for the population to exhibit its disease-treating effect on the subject. The term "effective dose" preferably refers to such an amount that a side effect can be suppressed to the minimum or within an allowable range while the disease-treating effect is exhibited on the subject. The dose of the gene-modified T cell population of the present invention to be administered to the subject may be appropriately designed in accordance with, for example, the condition, body weight, and age of the subject. According to, for example, the package insert of Kymriah infusion (Tisagenlecleucel) having an approval number of 23100FZX00002000, which is an approved drug for CAR-T cell therapy, when the gene-modified T cell population is used for relapsed or refractory CD19-positive B-cell acute lymphoblastic leukemia, the single dose of the gene-modified T cell population for a subject aged 25 or less (at the time of its administration) is from 0.2×10⁶ to 5.0×10⁶ (cells)/kg (body weight) in the case where the subject has a body weight of 50 kg or less, and the single dose is from 0.1×10⁸ to 2.5×10⁸ (cells)/kg (body weight) in the case where the subject has a body weight of more than 50 kg. The effective administration dose of the gene-modified T cell population of the present invention may follow the dose of the Kymriah infusion described above, but is not necessarily limited to the dose. The gene-modified T cell population of the present invention may be used alone for disease treatment, or may be used in combination with any other drug or therapy (e.g., chemotherapy).

### Examples

The present invention is described in more detail below by way of Examples. However, the present invention is by no means limited by these Examples and the like.

### Material and Method

### (1) Preparation of Retrovirus Solution

CAR-GPC1 included the leader sequence of human CD8α, an anti-GPC1 scFv, the extracellular domain/transmembrane domain/intracellular domain of human CD28, and the intracellular domain of CD3ζ. The base sequence (SEQ ID NO: 1; eLife, 2020, 9, e49392) of the CAR-GPC1 gene is illustrated in FIG. 1. A sequence encoding the anti-GPC1 scFV in SEQ ID NO: 1 is a base sequence encoding the heavy chain variable region and light chain variable region of an anti-GPC-1 antibody, the base sequence being produced from the base sequence information of a monoclonal anti-GPC-1 antibody (clone 1-12) (JP 6455935 B2) produced by using a chicken. The CAR-GPC1 gene was cloned in-frame into a retrovirus vector. The resultant recombinant retrovirus vector was transfected into G3T-hi cells together with a pGP vector and a pE-Eco vector. Thus, a retrovirus vector solution for infection was prepared. PG13 cells were infected with the retrovirus vector solution to produce producer cells, and a retrovirus solution was prepared from their culture supernatant.

### (2) T Cell Activation

### (2.1) Cells and Medium

Peripheral blood mononuclear cells (PBMCs) derived from a healthy donor (Normal Human PBMCs, Purified-Characterized; FUJIFILM Wako Pure Chemical Corporation, 93000-10M) were used. An AIM-V medium (Thermo Fisher Scientific, Inc., 0870112DK) containing a 10% human AB serum (Gemini BioProducts, Inc., 100-512), which had been heat-inactivated, and 500 IU/mL of interleukin-2 (Nipro Corporation, 87-890) was used as a medium for human PBMCs.

### (2.2) T Cell Activation with Free Anti-CD3 Antibody

The PBMCs of the section (2.1) were stimulated with 50 ng/mL of an anti-CD3 antibody (clone OKT3; Cell Science & Technology Institute, Inc., 6001T01) in the medium for human PBMCs for from 2 days to 8 days before gene introduction.

### (2.3) T Cell Activation with RetroNectin (trademark)-coated Plate and Free Anti-CD3 Antibody

The PBMCs of the section (2.1) were stimulated with 50 ng/mL of an anti-CD3 antibody (clone OKT3) on a RetroNectin (trademark)-coated plate (RetroNectin (trademark) Dish; Takara Bio Inc., T110A) containing the medium for human PBMCs for from 2 days to 8 days before the gene introduction.

### (2.4) T Cell Activation with T-activator CD3/CD28

The PBMCs of the section (2.1) were stimulated with anti-CD3 antibody-bound beads (Dynabeads Human T-Activator CD3/CD28; VERITAS Corporation, DB11131) in the medium for human PBMCs for from 2 days to 8 days before the gene introduction in accordance with their product protocol.

### (3) Gene Introduction with Virus-bound RetroNectin (trademark)-coated Plate (RN-Plate)-Plate Method

TheRetroNectin (trademark) (Takara Bio Inc., T100B) (1 mg/mL) 10 µL+D-PBS(-) (FUJIFILM Wako Pure Chemical Corporation, 049-29793) 400 µL was loaded into each well of 24-well transparent polystyrene flat-bottom untreated cell culture plate (Becton, Dickinson and Company, 351147), and was left at rest at 4°C overnight. Next day, the plate was washed with 1 mL of D-PBS(-), and then 500 µL/well of 3% BSA/D-PBS(-) was loaded thereinto. The plate was left at rest at room temperature for 30 minutes to prepare a RetroNectin (trademark)-coated plate. The retrovirus solution of the section (1) was placed on the RetroNectin (trademark)-coated plate, and was left at rest at 37°C and 5% CO₂ for from 5 hours to 6 hours so that its retrovirus was bound thereto. Next, the retrovirus solution was removed, and the cells activated and stimulated in the section (2) were seeded on the plate, followed by the gene introduction at 37°C and 5% CO₂. Next day, the medium for human PBMCs was added to the plate, and thereafter, expansion culture was performed while the medium was appropriately added thereto.

### (4) Gene Introduction with Virus-bound RetroNectin (trademark)-coated Magnetic Beads (RN-Beads)-Magnetic Bead Method

### (Coating of Magnetic Beads)

Magnosphere (trademark) MX200/Carboxyl (Medical & Biological Laboratories Co., Ltd., J-MS-X200C) was used as magnetic beads to each of which a protein was able to be bound. A required amount of the magnetic beads were washed with a 100 mM MES buffer twice by magnetic separation. Next, RetroNectin (trademark) was added at a ratio of 10 µg per 1 mg of the magnetic beads, and the mixture was incubated at room temperature overnight while being rotated at a low speed. RetroNectin (trademark) that had not been able to bind to the magnetic beads was removed by the magnetic separation of the beads, and the residue was incubated with D-PBS(-) containing 3% bovine serum albumin at room temperature for 30 minutes while being rotated at a low speed. Thus, RetroNectin (trademark)-coated magnetic beads were produced.

The resultant RetroNectin (trademark)-coated magnetic beads were dispersed in the retrovirus solution of the section (1), and the dispersed product was incubated at 32°C for 2 hours while being rotated at a low speed. Thus, the retrovirus was bound to each of the magnetic beads. After the retrovirus solution had been removed by magnetic separation, the total amount of the magnetic beads were seeded on a cell culture plate together with the cells activated and stimulated in the section (2), followed by the gene introduction at 37°C and 5% CO₂. Next day, the medium for human PBMCs was added to the plate, and thereafter, expansion culture was performed while the medium was appropriately added thereto.

### (5) Flow Cytometry Analysis

The cells that had been cultured for a sufficient time period were appropriately washed with D-PBS(-). With regard to the cells into each of which the gene had been introduced with the magnetic beads, the magnetic beads were removed from the culture in advance with a magnetic stand. Next, the washed product was appropriately suspended with D-PBS(-) containing 1% (w/v) bovine serum albumin, 2 mM EDTA, and 0.1 g/L anti-human IgG (Equitech-Bio Inc., SLH56). CAR-GPC1 and CCR7 expressed on the surface of each of the cells were stained with a 1/40 volume of each of a PE-labeled donkey anti-chicken IgY antibody (Jackson ImmunoResearch Inc., 703-116-155) (the antibody was bound to a chicken-derived anti-GPC1 scFv in CAR-GPC1) and an APC-labeled mouse anti-human CD197 (CCR7) antibody (BioLegend, Inc., 353214). The cells were left at rest at 4°C for 30 minutes, and then the supernatant of the cell suspension was removed by centrifugation. Next, the cells were washed with 10 volumes of D-PBS(-) twice. The cells after the washing were appropriately suspended in D-PBS(-) again, and CAR-GPC1-positive and CCR7-positive cells were evaluated with a flow cytometer (Becton, Dickinson and Company, Accuri (trademark) C6 Plus).

### (6) Amphipathic Molecule (Surfactant)

The following surfactants were each used as a surfactant added at the time of the gene introduction.
·Poly(ethylene glycol)-block-poly(propylene glycol)-block-poly(ethylene glycol) (FUJIFILM Wako Pure Chemical Corporation, 592-18721); 10% Pluronic (trademark) F68, in which the average molecular weight of its (PO) unit is about 1,750, and the content ratio (molecular weight ratio) of its (EO) unit is about 80%.
·Poly(ethylene glycol)-block-poly(propylene glycol)-block-poly(ethylene glycol) (Sigma Corporation, 542342-250G); Pluronic (trademark) F108, in which the average molecular weight of its (PO) unit is about 3,250, and the content ratio (molecular weight ratio) of its (EO) unit is about 80%.
·Poly(ethylene glycol)-block-poly(propylene glycol)-block-poly(ethylene glycol) (Sigma Corporation, P2443-250G); Pluronic (trademark) F127, in which the average molecular weight of its (PO) unit is about 4,000, and the content ratio (molecular weight ratio) of its (EO) unit is about 70%.
·Poly(ethylene glycol)-block-poly(propylene glycol)-block-poly(ethylene glycol) (Sigma Corporation, 435406-250ML); Pluronic (trademark) L31, in which the average molecular weight of its (PO) unit is about 950, and the content ratio (molecular weight ratio) of its (EO) unit is about 10%.
·Poly(ethylene glycol)-block-poly(propylene glycol)-block-poly(ethylene glycol) (Sigma Corporation, 435414-250ML); Pluronic (trademark) L35, in which the average molecular weight of its (PO) unit is about 950, and the content ratio (molecular weight ratio) of its (EO) unit is about 50%.
·Poly(ethylene glycol)-block-poly(propylene glycol)-block-poly(ethylene glycol) (Sigma Corporation, 435449-250ML); Pluronic (trademark) L64, in which the average molecular weight of its (PO) unit is about 1,750, and the content ratio (molecular weight ratio) of its (EO) unit is about 40%.
·Poly(ethylene glycol)-block-poly(propylene glycol)-block-poly(ethylene glycol) (Sigma Corporation, 435457-250ML); Pluronic (trademark) L121, in which the average molecular weight of its (PO) unit is about 4,000, and the content ratio (molecular weight ratio) of its (EO) unit is about 10%.
·Poly(ethylene glycol)-block-poly(propylene glycol)-block-poly(ethylene glycol) (Sigma Corporation, 435465-250ML); Pluronic (trademark) P123, in which the average molecular weight of its (PO) unit is about 4,000, and the content ratio (molecular weight ratio) of its (EO) unit is about 30%.
·10 w/v% polyoxyethylene (20) sorbitan monolaurate solution (FUJIFILM Wako Pure Chemical Corporation, 161-24801); 10% Tween (trademark) 20.
·Polyoxyethylene (10) octyl phenyl ether (FUJIFILM Wako Pure Chemical Corporation, 160-24751); Triton (trademark) X100.
·Nonidet (trademark) P40 (Sigma Corporation, 21-3277-2).

### Experimental Example 1

### (Improvement in Gene Introduction Efficiency by Magnetic Bead Method)

T cells were activated in accordance with the methods (1) to (4) by the stimulation of PBMCs with an anti-CD3 antibody (method of the section (2.2)). 2 Days after that, a CAR-GPC1 gene was introduced into each of the cells by each of the plate method (gene introduction with a RN-Plate in the method (3)) and the magnetic bead method (gene introduction with RN-Beads in the method (4)), and the cells were cultured. On the 7th day after the gene introduction, the cells were collected, and the expression ratio of CAR-GPC1 was examined by detecting the cells each having CAR-GPC1 expressed on its surface through flow cytometry analysis in accordance with the method (5).

The results are shown in FIG. 2. As a result of the verification of the expression ratio of CAR-GPC1, in the plate method, a CAR-GPC1-positive ratio was 12%. In contrast, in the magnetic bead method, a T cell activation period was 45%, and hence the improving effect of the magnetic bead method on gene introduction efficiency was observed.

### Experimental Example 2

### (Investigation of PBMC Activation Method in Magnetic Bead Method)

PBMCs were activated by any one of the methods (2.2) to (2.4) (T cell activation with a free anti-CD3 antibody, T cell activation with a RetroNectin (trademark)-coated plate and the free anti-CD3 antibody, or T cell activation with Human T-Activator CD3/CD28) on a scale 100 times as large as that of Experimental Example 1. A CAR-GPC1 gene was introduced into each of the PBMCs, which had been activated for 8 days, in accordance with the magnetic bead method in the method (4), and the cells were cultured. A culture bag CultiLife 215 (Takara Bio Inc., FU0005) was used as an incubator instead of the cell culture plate. On the 5th day after the gene introduction, the cells were collected, and the expression ratio of CAR-GPC1 was examined through flow cytometry analysis in accordance with the method (5).

The results are shown in FIG. 3. As a result of the verification of the expression ratio of CAR-GPC1, in the T cell activation with the free anti-CD3 antibody (Anti CD3 Ab), the expression ratio was 54%, in the T cell activation with the RetroNectin (trademark)-coated plate and the free anti-CD3 antibody (Anti CD3 Ab/RN), the expression ratio was 54%, and in the T cell activation with the Human T-Activator CD3/CD28 (Human T-Activator CD3/CD28), the expression ratio was 26%. It was revealed from the foregoing that the combination of the gene introduction by the magnetic bead method with the activation with the free anti-CD3 antibody was efficient.

### Experimental Example 3

### (Improvement in Gene Introduction Efficiency by Amphipathic Molecule)

A CAR-GPC1 gene was introduced into each of PBMCs, which had been subjected to T cell activation by the method (2.2), in accordance with the magnetic bead method in the method (4) 8 days after the activation. When the magnetic beads and the cells were caused to react with each other in the gene introduction, one kind of amphipathic molecule out of Pluronic (trademark) L31, Pluronic (trademark) L35, Tween 20 (trademark), Triton (trademark) X100, and Nonidet (trademark) P40 was added to the medium so that its final concentration became 0.01 mass%, 0.05 mass%, or 0.1 mass%. A medium containing the same kind of amphipathic molecule at the same concentration was similarly used in the subsequent medium exchange. On the 7th day after the gene introduction, the cells were collected, and the expression ratio of CAR-GPC1 was examined through flow cytometry analysis in accordance with the method (5).

The results are shown in FIG. 4. When no amphipathic molecules were added (No additives), the expression ratio of CAR-GPC1 was 26%. In contrast, when Pluronic (trademark) L35 was added, the expression ratio was from 32% to 39%, and when 0.01% of Tween 20 (trademark) was added, the expression ratio was 31%. Pluronic (trademark) L35 and a low concentration of Tween 20 (trademark) each showed a gene introduction efficiency-improving effect. Pluronic (trademark) L31 showed no gene introduction efficiency-improving effect. In addition, when each of Triton (trademark) X100, NP-40, 0.05% and 0.1% of Tween 20 (trademark), and 0.1% of Pluronic (trademark) L31 was added, the expression ratio of CAR-GPC1 could not be evaluated because the cells were died.

### Experimental Example 4

### (Improvement in Gene Introduction Efficiency by Amphipathic Molecule 2)

Gene-introduced cells were prepared under the same conditions as those of Experimental Example 3 except that the kind of the amphipathic molecule was changed, followed by the examination of the expression ratio of CAR-GPC1 through flow cytometry analysis. One kind of Pluronic (trademark) F68 or Pluronic (trademark) F127 was used as the amphipathic molecule. Pluronic (trademark) F68 was added to the medium so that its final concentration became 0.01%, 0.03%, or 0.10%. Pluronic (trademark) F127 was added to the medium so that its final concentration became 0.05%, 0.10%, or 0.20%.

The results are shown in FIG. 5. When no amphipathic molecules were added (No additives), the expression ratio of CAR-GPC1 was 54%. In contrast, when Pluronic (trademark) F68 was added, the expression ratio was from 61% to 66%, and when Pluronic (trademark) F127 was added, the expression ratio was 63% to 71%. Pluronic (trademark) F68 or Pluronic (trademark) F127 showed a gene introduction efficiency-improving effect.

### Experimental Example 5

### (Yield of CCR7-positive T Cells in Magnetic Bead Method-Comparison among PBMC Activation Methods)

The PBMCs subjected to the activation and the gene introduction in Experimental Example 2 were collected on the 5th day after the gene introduction, and the ratios of CAR+ and CCR7+ cells on the surfaces of the cells were examined through flow cytometry analysis in accordance with the method (5).

The results are shown in FIG. 6. The ratio of CAR-GPC1-positive (CAR+) and CCR7-positive (CCR7+) cells was 19% in the T cell activation with the free anti-CD3 antibody (Anti CD3 Ab), was 21% in the T cell activation with the RetroNectin (trademark)-coated plate and the free anti-CD3 antibody (Anti CD3 Ab/RN), and was 10% in the T cell activation with the Human T-Activator CD3/CD28 (Human T-Activator CD3/CD28). In addition, the ratios of the entire CAR-GPC1-positive (CAR+) cells were 54%, 54%, and 26%, respectively. It was revealed from the foregoing that the combination of the gene introduction method including using the magnetic beads with the activation with the free anti-CD3 antibody improved not only gene introduction efficiency but also the yield of CAR-GPC1-expressing T cells maintaining their undifferentiated states.

### Experimental Example 6

### (Yield of CCR7-positive T Cells-Comparison between Gene Introduction Methods)

The CAR-GPC1 gene was introduced into each of cells, which had been activated with the free anti-CD3 antibody in accordance with the method (2.2), by the plate method in the method (3) or the magnetic bead method in the method (4) 4 days after the activation. On the 7th day after the gene introduction, the cells were collected, and the ratios of CAR+ and CCR7+ cells were examined through flow cytometry analysis in accordance with the method (5).

The results are shown in FIG. 7. While the ratio of CAR+/CCR7+ cells was 6% in the plate method (RN-Plate), the ratio was 30% in the magnetic bead method (RN-Beads). In addition, the ratios of the entire CAR-GPC1-positive (CAR+) cells were 12% and 60%, respectively. It was revealed from the foregoing that the magnetic bead method was superior to the plate method not only in gene introduction efficiency but also in improvement in yield of CAR-GPC1-expressing T cells maintaining their undifferentiated states.

### Experimental Example 7

### (Yield of CCR7-positive T Cells-Influences of Amphipathic Molecules)

The CAR-GPC1 gene was introduced into each of cells, which had been activated with the free anti-CD3 antibody in accordance with the method (2.2), by the magnetic bead method in the method (4) 4 days after the activation. At the time of the gene introduction, one kind of amphipathic molecule out of Pluronic (trademark) L64, Pluronic (trademark) L121, Pluronic (trademark) P123, Pluronic (trademark) F68, and Pluronic (trademark) F108 was added to the medium so that its final concentration became 0.01%, 0.05%, or 0.1%. A medium containing the same kind of amphipathic molecule at the same concentration was similarly used in the subsequent medium exchange. On the 7th day after the gene introduction, the cells were collected, and the ratios of CAR+ and CCR7+ cells were examined through flow cytometry analysis in accordance with the method (5).

The results are shown in FIG. 8. When no amphipathic molecules were added (No additives), the ratio of CAR+/CCR7+ cells was 30%. In contrast, when Pluronic (trademark) F68 or Pluronic (trademark) F108 was added, the ratio was from 31% to 38%. In addition, in the case of "No additives," the ratio of the CCR7+ cells in the CAR+ cells was 50%. In contrast, when Pluronic (trademark) F68 or Pluronic (trademark) F108 was added, the ratio was from 52% to 66%. It was recognized from the foregoing that Pluronic (trademark) F68 and Pluronic (trademark) F108 each had an increasing effect on the yield of the CCR7-positive cells. Meanwhile, in each of the media having added thereto Pluronic (trademark) L64, Pluronic (trademark) L121, and Pluronic (trademark) P123, the expression ratio of CAR-GPC1 could not be evaluated because the cells were died.

### Experimental Example 8

### (Yield of CAR+ Cells-Influences of Amphipathic Molecules)

The CAR-GPC1 gene was introduced into each of cells, which had been activated with the free anti-CD3 antibody in accordance with the method (2.2), by the plate method in the method (3) or the magnetic bead method in the method (4) 5 days after the activation. At the time of the gene introduction, a medium having added thereto no amphipathic molecules was used in the plate method, and a medium containing 0% (No additives), 0.01%, 0.05%, or 0.1% of Pluronic (trademark) F68 was used in the magnetic bead method. A medium containing the same kind of amphipathic molecule at the same concentration was also used in the subsequent medium exchange. On the 7th day after the gene introduction, the magnetic beads were removed, and the cells were collected, followed by the examination of the ratio of CAR-GPC1-positive cells through flow cytometry analysis in accordance with the method (5). Further, part of the collected cells were subjected to expansion culture in a medium free of Pluronic (trademark) F68 for 1 week. After that, the number of the CAR-GPC1-positive cells and the ratio thereof in all the cells were examined through flow cytometry by the same procedure.

The results are shown in FIGS. 9. In the magnetic bead method, the ratio (FIG. 9A) and number (FIG. 9B) of the CAR-GPC1-positive cells each increased as compared to those in the plate method. It was recognized from those results that even after the removal of the magnetic beads, the cells proliferated while a positive ratio was maintained. Further, when the gene was introduced in a medium containing Pluronic (trademark) F68 in the magnetic bead method, a tendency in that the ratio of the CAR-GPC1-positive cells increased was observed (FIG. 9A).

### Experimental Example 9

### (Shortening Effect on CAR-T Cell Production Period in Magnetic Bead Method)

The shortening effect on a CAR-GPC1-positive cell production period in the magnetic bead method was evaluated by calculating, with respect to the number of the CAR-GPC1-positive cells obtained on the 7th day of the culture in the plate method of Experimental Example 8, the number of culture days in which the same number of the cells were obtained by the magnetic bead method. As shown in FIG. 10, cell numbers on the 1st day to 7th day of the culture under the respective conditions of Experimental Example 8 were estimated on the basis of the ratios and numbers of the CAR-GPC1-positive cells on the 1st day and 7th day of the culture, and a doubling time calculated therefrom, and a culture period required to obtain, in the magnetic bead method, the number of the CAR-GPC1-positive cells corresponding to that on the 7th day of the culture in the plate method was calculated. FIG. 10 shows a change in number of the CAR-GPC1-positive cells with the number of the culture days. As a result, the following trial calculation was made, though the number of days varied depending on the addition amount of Pluronic (trademark) F68: a required number of the CAR-GPC1-positive cells was reached at the time point of from the 4th day to the 7th day from the day of the gene introduction. It was suggested from the foregoing that in the magnetic bead method, a time period until the required number of the CAR-GPC1-positive cells were obtained was able to be shortened by up to about 3 days as compared to the plate method.

When reference is made to the case of Kymriah infusion, the number of CAR-T cells to be administered in the treatment of B-cell acute lymphoblastic leukemia is from 0.2×10⁶ to 5.0×10⁶ (cells)/kg (body weight) in the case of a patient having a body weight of 50 kg or less, and is from 0.1×10⁸ to 2.5×10⁸ (cells)/kg (body weight) in the case of a patient having a body weight of more than 50 kg
([www.pmda.go.jp/regenerative_medicines/2019/R20190423001/300242000_2310 0FZX00002000_B100_1.pdf]).
To produce 2.5×10⁸ cells, a scale-up by a factor of 1,700 is required in the plate method. In consideration of scale-up culture for clinical applications, the significance of obtaining a production period-shortening effect of up to 3 days may be large.

## Claims

1. A production method of a gene-modified T cell population, comprising
mixing a cell population containing T cells with beads each having bound thereto a virus containing a target gene, and introducing the target gene into each of the cells of the cell population,
wherein the cell population containing the T cells is cultured in a solution containing a CD3 signal activator that is present without being immobilized on a solid phase.

2. The production method according to claim 1, wherein the cell population containing the T cells is whole blood, peripheral blood mononuclear cells, a cell population in a buffy coat solution, a cell population obtained by leukapheresis, or a culture thereof.

3. The production method according to claim 1, wherein the CD3 signal activator is an anti-CD3 antibody.

4. The production method according to claim 3, wherein the anti-CD3 antibody is OKT3.

5. The production method according to claim 1, wherein the solution containing the CD3 signal activator contains IL-2.

6. The production method according to claim 1, wherein the virus is at least one kind selected from the group consisting of: a retrovirus; a lentivirus; an adenovirus; an adeno-associated virus; and a simian virus.

7. The production method according to claim 1, wherein the beads are each coated with a molecule having abilities to bind to the T cells and the virus.

8. The production method according to claim 7, wherein the molecule having abilities to bind to the T cells and the virus is at least one kind selected from the group consisting of: gelatin; collagen; elastin; fibronectin; pronectin; laminin; tenascin; fibrin; fibroin; entactin; thrombospondin; fragments thereof; variants thereof; and derivatives thereof.

9. The production method according to claim 7, wherein the molecule having abilities to bind to the T cells and the virus is fibronectin, or is a fibronectin fragment or a fibronectin variant having a cell-adhesion domain and a heparin-binding domain of fibronectin.

10. The production method according to claim 1, wherein the beads are magnetic beads.

11. The production method according to claim 1, wherein the CD3 signal activator is free from being bound to the beads.

12. The production method according to claim 1, wherein the mixing of the cell population with the beads each having bound thereto the virus is performed in the presence of a polyoxyethylene-polyoxypropylene (POE/POP) triblock copolymer in which a content of a polyoxyethylene (EO) unit is 50% or more, and a polyoxypropylene (PO) unit has an average molecular weight of 900 or more.

13. The production method according to claim 12, wherein in the polyoxyethylene-polyoxypropylene (POE/POP) triblock copolymer, the (PO) unit has an average molecular weight of from 1,700 to 4,500, and the content of the (EO) unit is 50% or more.

14. The production method according to claim 12, wherein in the polyoxyethylene-polyoxypropylene (POE/POP) triblock copolymer, the (PO) unit has an average molecular weight of from 1,700 to 4,500, and the content of the (EO) unit is 70% or more.

15. The production method according to claim 1, wherein the target gene is a gene encoding a chimeric antigen receptor.

16. The production method according to claim 1, further comprising culturing the cells each having introduced thereinto the target gene to provide the gene-modified T cell population.

17. The production method according to claim 16, wherein a time period from culturing of the cell population containing the T cells in the solution containing the CD3 signal activator to providing the gene-modified T cell population by the culturing the cells each having introduced thereinto the target gene is from 6 days to 21 days.

18. The production method according to claim 16, further comprising recognizing, on cells of the gene-modified T cell population, expression of at least one cell surface antigen selected from the group consisting of: CD45RA; CCR7; CD62L; CD73; CXCR3; CD27; CD28; KLRG1; IL7R; BACH2; TCF7; ID3; LEF1; FOXP1; KLF7; CD45RO; and CD95.

19. A gene-modified T cell population, which is produced by the method of claim 1.

20. The gene-modified T cell population according to claim 19, wherein the gene-modified T cell population expresses at least one cell surface antigen selected from the group consisting of: CD45RA; CCR7; CD62L; CD73; CXCR3; CD27; CD28; KLRG1; IL7R; BACH2; TCF7; ID3; LEF1; FOXP1; KLF7; CD45RO; and CD95.

21. A pharmaceutical composition for a cancer or an immune disease, comprising the gene-modified T cell population of claim 19 as an active ingredient.

22. Use of the gene-modified T cell population of claim 19 in production of a pharmaceutical composition for a cancer or an immune disease.

23. The gene-modified T cell population according to claim 19, which is for use in the treatment of a cancer or an immune disease.

24. A method of treating a cancer or an immune disease in a subject in need thereof, comprising a step of administering the gene-modified T cell population of claim 19 to the subject.
